# EUROPEAN PATENT APPLICATION

(11) **EP 4 420 696 A1**
(43) Date of publication of application: **28.08.2024**
(21) Application number: 23305253.9
(22) Date of filing: 24.02.2023
(51) Int. Cl.: A61M 5/28, A61M 5/20, A61M 5/315, A61M 5/32, A61M 5/24, A61M 5/31

(54) **CASSETTE BODY CLIP FOR AUTO-INJECTOR**

(71) Applicant: Becton, Dickinson and Company, Franklin Lakes, NJ 07417-1880 (US)
(72) Inventor: BESSON, Nicolas, 38650 Treffort (FR); PLOUVIER, Adrien, 38400 SAINT MARTIN D'HERES (FR); ALVAIN, Olivier, 38180 SEYSSINS (FR)
(74) Representative: Germain Maureau

(57) **Abstract**

A drug delivery device includes an upper housing shell, a lower housing shell, and a syringe. The syringe includes a barrel, a stopper, and a needle, at least a portion of the syringe positioned within the lower housing shell. The drug delivery device further includes a drive assembly including a plunger body configured to move the stopper within the barrel upon actuation of the drive assembly, a cassette body configured to receive the syringe, and a motor body configured to engage the cassette body. The cassette body includes a pair of sidewalls and at least one cassette body clip extending from the sidewalls and configured to engage a lower shell clip of the lower shell housing.

## Description

### BACKGROUND OF THE INVENTION

### Field of the Invention

The present disclosure relates generally to a drug delivery device and, more specifically, to an auto-injector.

### Description of Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. Many of these devices, such as auto-injectors, are designed so that the reservoir, such as a pre-filled syringe, is assembled into the device during assembly of the device. In addition to automatically deploying the needle-injection mechanism, many drug delivery devices also automatically shield the needle after use of the device to prevent any unintended contact with the needle.

Automatic injection devices include several interlocking parts that must be assembled in a precise manner in order for the devices to function effectively as intended. Poor assembly can lead to component failure, unintended movement or disconnection of components relative to each other, and other adverse effects that ultimately result in reduced efficiency and/or reliability of the device.

### SUMMARY OF THE INVENTION

Provided herein is a drug delivery device including an upper housing shell, a lower housing shell, and a syringe. The syringe includes a barrel, a stopper, and a needle, at least a portion of the syringe positioned within the lower housing shell. The drug delivery device further includes a drive assembly including a plunger body configured to move the stopper within the barrel upon actuation of the drive assembly, a cassette body configured to receive the syringe, and a motor body configured to engage the cassette body. The cassette body includes a pair of sidewalls and at least one cassette body clip extending from the sidewalls and configured to engage a lower shell clip of the lower shell housing

In some embodiments, the at least one cassette body clip includes an orthogonal proximal face extending outward from the sidewall in a direction substantially perpendicular to a longitudinal axis of the drug delivery device.

In some embodiments, the at least one cassette body clip includes a ramped leading face configured to engage the lower shell clip during assembly of the cassette body into the lower housing shell.

In some embodiments, each of the sidewalls defines a pocket proximally of the at least one cassette body clip into which the lower shell clip at least partially extends when the cassette body is fully inserted into the lower housing shell.

In some embodiments, the lower shell clip extends inward from the lower housing shell toward the cassette body.

In some embodiments, the lower shell clip includes a hinge integrally formed with the lower housing shell.

In some embodiments, the lower shell clip extends distally from the hinge and is rotatable about the hinge.

In some embodiments, the lower shell clip is configured to engage the orthogonal proximal face to prevent withdrawal of the cassette body from the lower housing shell.

In some embodiments, the lower housing shell includes a rigid wall configured to limit inward deflection of the sidewall of the cassette body.

In some embodiments, with a withdrawal force is applied to the cassette body, the lower shell clip is configured to deflect the cassette body clip inward until the cassette body clip contacts the rigid wall.

In some embodiments, the at least one cassette body clip is configured to not contact the rigid wall during insertion of the cassette body into the lower housing shell.

In some embodiments, the rigid wall includes a gusset connecting the rigid wall to an orifice wall at least partially surrounding a needle shield of the syringe.

In some embodiments, with the cassette body assembled with the lower housing shell, a gap is defined between the orthogonal proximal face of the cassette body clip and a distal end of the lower shell clip.

In some embodiments, with the cassette body assembled with the lower housing shell, a gap is defined between the pocket and the lower shell clip.

In some embodiments, the lower shell clip is less rigid than the cassette body clip.

These and other features and characteristics of drug delivery devices and methods of operation and functions of the related elements of structures and the combination of parts and economies of manufacture, will become more apparent upon consideration of the following description and the appended claims with reference to the accompanying drawings, all of which form a part of this specification. It is to be expressly understood, however, that the drawings are for the purpose of illustration and description only.

### BRIEF DESCRIPTION OF THE DRAWINGS

FIG. 1 is a perspective view of a drug delivery device according to non-limiting embodiments described herein;
FIG. 2 is a cross-sectional view of the drug delivery device of FIG. 1;
FIG. 3 is a perspective view of a second subassembly of the drug delivery device of FIG. 1;
FIG. 4 is a perspective view of a first subassembly of the drug delivery device of FIG. 1;
FIG. 5 is a perspective view of a motor body of the drug delivery device of FIG. 1;
FIG. 6 is a detail perspective view of the motor body of FIG. 5;
FIG. 7 is a top perspective view of a cassette body of the drug delivery device of FIG. 1;
FIG. 8 is a bottom perspective view of the cassette body of FIG. 7;
FIG. 9 is a detail cross-sectional view of a distal end of the drug delivery device of FIG. 1;
FIG. 10 is a detail perspective view of a cassette body clip of the cassette body of FIG. 7;
FIG. 11 is a detail perspective view of a lower shell clip of the drug delivery device of FIG. 1;
FIG. 12 is a detail cross-sectional view of the distal end of the drug delivery device of FIG. 1 during connection of the cassette body to the lower housing shell;
FIG. 13 is a detail cross-sectional view of the distal end of the drug delivery device of FIG. 1 during connection of the cassette body to the lower housing shell;
FIG. 14 is a detail cross-sectional view of the distal end of the drug delivery device of FIG. 1 during attempted withdrawal of the cassette body from the lower housing shell; and
FIG. 15 is a detail perspective view of the distal end of the drug delivery device of FIG. 1 during connection of the cassette body to the lower housing shell.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of " 1 to 10" is intended to include all sub-ranges between (and including) the recited minimum value of 1 and the recited maximum value of 10, that is, having a minimum value equal to or greater than 1 and a maximum value of equal to or less than 10.

When used in relation to a component of a drug delivery device, the term "distal" refers to a portion of said component nearest to a patient. When used in relation to a component of a drug delivery device, the term "proximal" refers to a portion of said component farthest from the patient.

As used herein, the term "at least one of" is synonymous with "one or more of'. For example, the phrase "at least one of A, B, and C" means any one of A, B, and C, or any combination of any two or more of A, B, and C. For example, "at least one of A, B, and C" includes one or more of A alone; or one or more of B alone; or one or more of C alone; or one or more of A and one or more of B; or one or more of A and one or more of C; or one or more of B and one or more of C; or one or more of all of A, B, and C. Similarly, as used herein, the term "at least two of' is synonymous with "two or more of'. For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

International Patent Application Publication Nos. WO 2020/173991, WO 2020/173992, WO 2020/173993, WO 2020/173994, and WO 2020/173995 are incorporated herein by reference in their entirety.

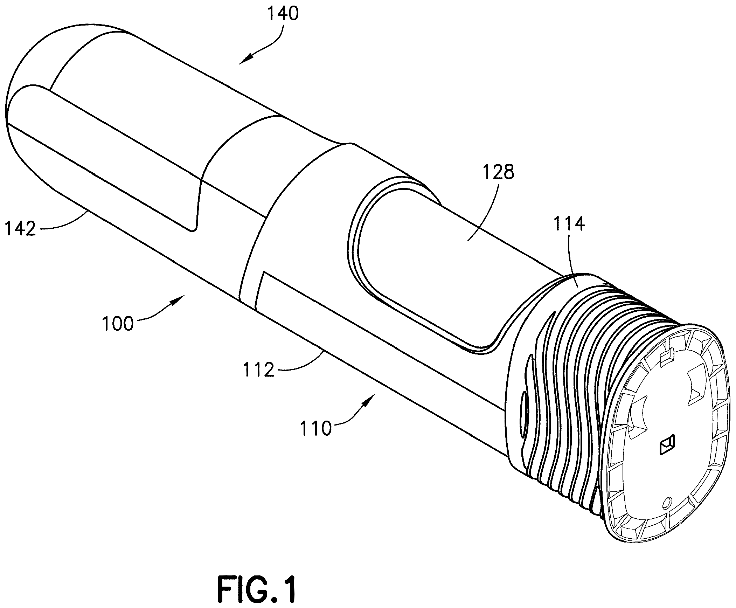

Turning to FIGS. 1-4, shown is a drug delivery device 100. The drug delivery device 100 includes a first subassembly 110, a second subassembly 140, and a syringe 116. The first subassembly 110 may include a lower housing shell 112, a cap 114, a syringe holder 118, a needle cover 124, and a cassette body 128. The second subassembly 140 may include a drive assembly including a plunger body 150. The plunger body 150 includes a plunger rod 152 and a spring guide member 154 including a drive member 156 and a drive opening 158. The second subassembly 140 may further include a motor body 159, a lever actuation member 130, and an upper housing shell 142. The syringe 116 may be received by the syringe holder 118 and includes a barrel 117, a stopper 122, a needle 120, and a rigid needle shield (RNS) 126. Although an RNS is utilized in the example shown in the accompanying drawings, other suitable needle shield arrangements may be utilized. The lower housing shell 112, the cassette body 128, and the upper housing shell 142 generally form a housing for receiving the various components of the drug delivery device 100, although other suitable housing arrangements may be utilized. The first subassembly 110 and the second subassembly 140 may be secured to each other during assembly by a locking clip, although other suitable arrangements may be utilized. The upper housing shell 142 includes an outer lip 143 configured to fit around an inner lip 113 of the lower housing shell 112 when the device 100 is assembled. The drug delivery device 100 may be an auto-injector, although the features described herein may be incorporated into other suitable drug delivery devices.

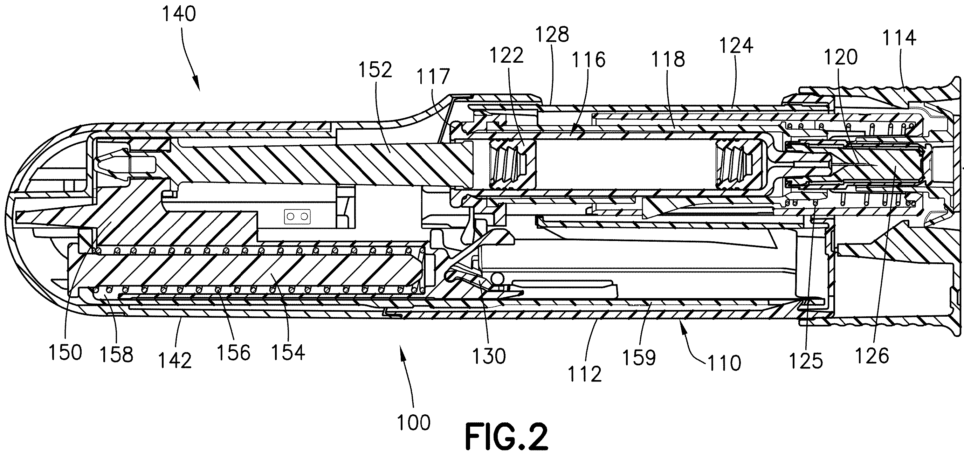

With continued reference to FIG. 2, the drive member 156 may be a compression spring received within a drive opening 158 defined by the plunger body 150, although other suitable drive members may be utilized, including, but not limited to, compressed gas, an electric motor, hydraulic pressure, other types of springs, etc. The drive member 156 engages the plunger body 150 and the motor body 159 and biases the plunger body 150 in a direction extending from the second subassembly 140 toward the first subassembly 110. The plunger body 150 defines a lever opening that receives the lever actuation member 130 and defines the rotation axis of the lever actuation member 130. The lever actuation member 130 prevents movement of the plunger body 150 when the lever actuation member 130 is in the locked position through engagement of the lever actuation member 130 with the motor body 159. Upon rotation of the lever actuation member 130 from the locked position to the released position, the lever actuation member 130 is disengaged from the motor body 159 thereby allowing the drive member 156 to move the plunger body 150 and the plunger rod 152 toward the first subassembly 110. The plunger rod 152 and the drive member 156 are spaced from and parallel to each other and extend in a longitudinal direction of the device 100.

The spring guide member 154 is secured to the upper housing shell 142 and received within the drive opening 158 of the plunger body 150. The drive member 156 is received by the spring guide member 154 such that the drive member 156 is positioned between the plunger body 150 and the spring guide member 154. In some embodiments, the drive assembly may also include a plunger rod cover (not shown) that receives the plunger rod 152 of the plunger body 150. The plunger rod cover may be configured to guide insertion of the plunger rod 152 into the barrel 117 of the syringe 116 and engage the stopper 122 to dispense the medicament from the barrel 117 of the syringe 116.

The drug delivery device 100 is configured to automatically deliver a dose of medicament from the syringe 116 to a patient upon actuation of the device 100. More specifically, upon actuation of the drug delivery device 100, the drive assembly is configured to engage the stopper 122 of the syringe 116, displace the syringe 116 such that the needle 120 pierces the skin of the patient, and displace the stopper 122 within the barrel 117 of the syringe 116 to deliver the medicament within the barrel 117. The drug delivery device 100 includes a storage position, a pre-use position, an actuation position, an injection position, and a post-use position, as described in International Patent Application Publication Nos. WO 2020/173991, WO 2020/173992, WO 2020/173993, WO 2020/173994, and WO 2020/173995.

The needle cover 124 is configured to shield the needle 120 of the syringe 116 from the patient when the device 100 is in the pre-use and the post-use positions. In particular, the needle cover 124 is moveable between a pre-use position, an actuation position, and a post-use position, with a spring 125 biasing the needle cover 124 towards the pre-use position and the post-use position. The spring 125 may be positioned between the needle cover 124 and the syringe holder 118, although other suitable arrangements may be utilized.

The lever actuation member 130 is moveable between a locked position where movement of the drive assembly is prevented and a released position where movement of the drive assembly is allowed. More specifically, the lever actuation member 130 is rotatable about a rotation axis between the locked position and the released position. When the lever actuation member 130 is in the locked position, the lever actuation member 130 is engaged with the motor body 159 and the drive assembly to prevent movement of the drive assembly.

When the lever actuation member 130 is in the released position, which may be achieved by pressing the needle cover 124 onto the patient's skin at the site of injection, shifting the needle cover proximally into the lower housing shell 112, the lever actuation member 130 is disengaged from the motor body 159 thereby allowing movement of the drive assembly toward the syringe 116. The rotation axis of the lever actuation member 130 extends perpendicular to a longitudinal axis of the device 100, although other suitable arrangements may be utilized.

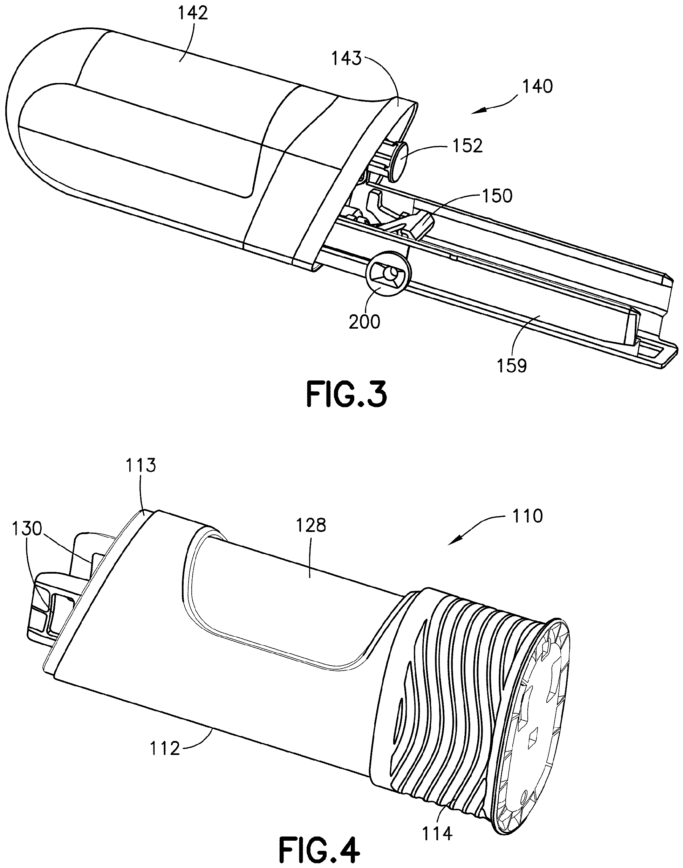

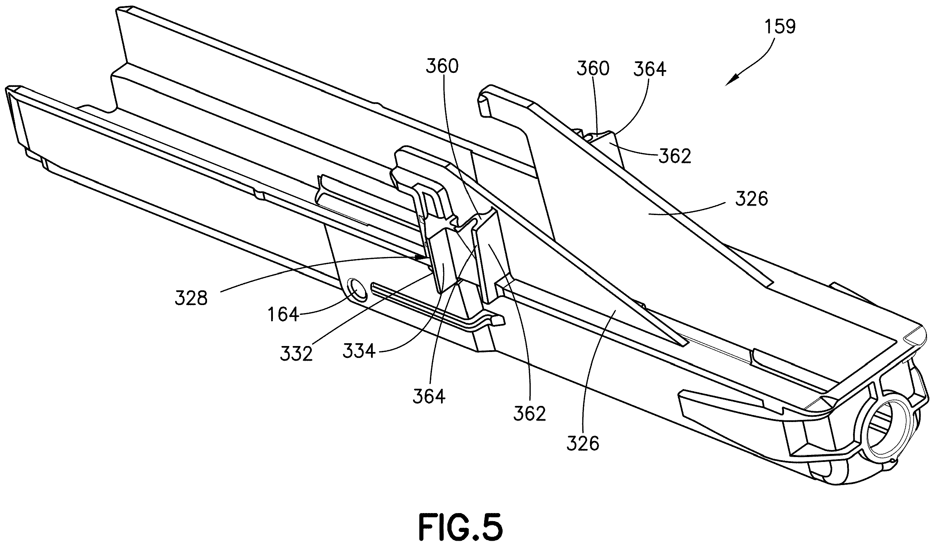

FIGS. 3 and 4 illustrate the second subassembly 140 and the first subassembly 110, respectively, as these subassemblies may be provided for final assembly of the device 100. As shown in FIG. 3, a safety pin 200 is inserted through apertures 164 (see FIG. 5) of the motor body 159. The apertures 164 are located in proximity to a distal end of the plunger body 150 so that with the safety pin 200 inserted through the apertures 164, the safety pin 200 prevents rotation of the lever actuation member 130 and unintentional actuation of the drive member 156. The safety pin 200 is removed prior to or during final assembly of the device 100.

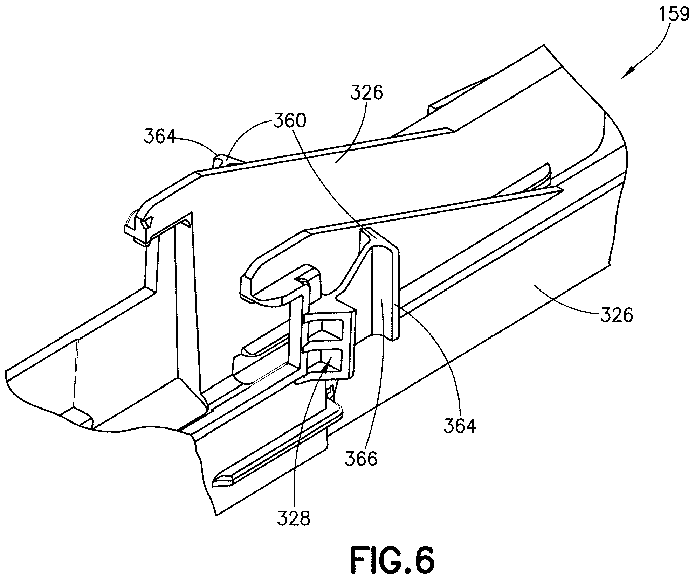

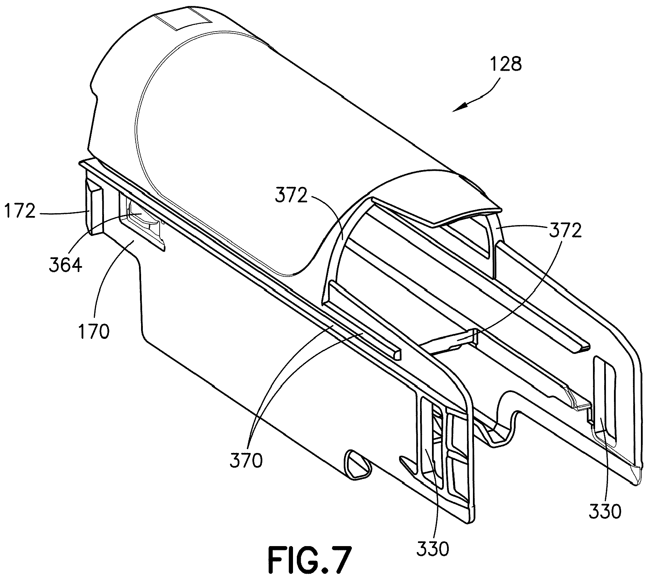

Referring now to FIGS. 5 and 6, the motor body 159 includes a pair of opposing sidewalls 326 configured to receive the plunger body 150 therebetween. The motor body 159 defines the safety pin apertures 164 extending transversely through the sidewalls 326 to facilitate insertion of the safety pin 200 (see FIG. 3). The motor body 159 further includes at least one cassette clip 328 extending from the respective sidewalls 326 for engaging complementary openings 330 (see FIGS. 7-8) defined by the cassette body 128. Engagement of the at least one cassette clip 328 with the openings 330 secures the motor body 159 to the cassette body 128. The at least one cassette clip 328 includes an angled face 332 and a planar face 334, which are configured to allow insertion of the at least one cassette clip 328 into the corresponding openings 330 of the cassette body 128, but prevent easy removal of the cassette clips 328 once inserted into the openings 330 of the cassette body 128.

The motor body 159 is configured to be inserted in the upper housing shell 142 of the second subassembly 140. The motor body 159 includes at least one shell stop 360 extending outward from the opposing sidewalls 326 of the motor body 159. Each shell stop 360 has a planar proximal face 362 configured to engage an internal structure of the upper housing shell 142 to index the motor body 159 in the upper housing shell 142. The at least one shell stop 360 extends a sufficient distance outward from the sidewalls 326 of motor body 159 so that the at least one shell stop 360 remains at least partially engaged with the corresponding internal structure of the upper housing shell 142 when the sidewalls 326 of the motor body 159 are deflected inward during connection of the motor body 159 to the cassette body 128. In particular, each of the at least one shell stops 360 includes a cantilevered end 364. The cantilevered end 364 is reinforced by a radiused edge 366 to prevent failure of the shell stop 360 due to load applied to the cantilevered end 364. For example, the radiused edge 366 may be sized to prevent breakage of the at least one shell stop 360 if the device 100 is dropped during handling.

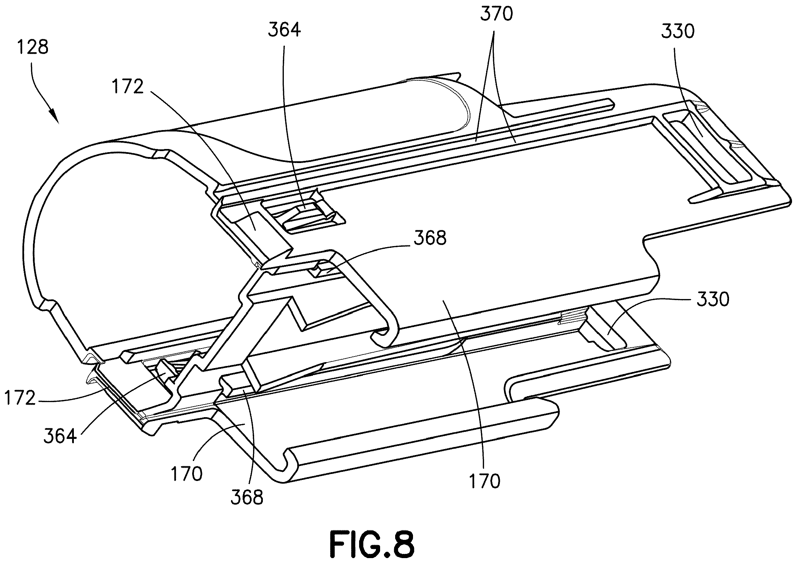

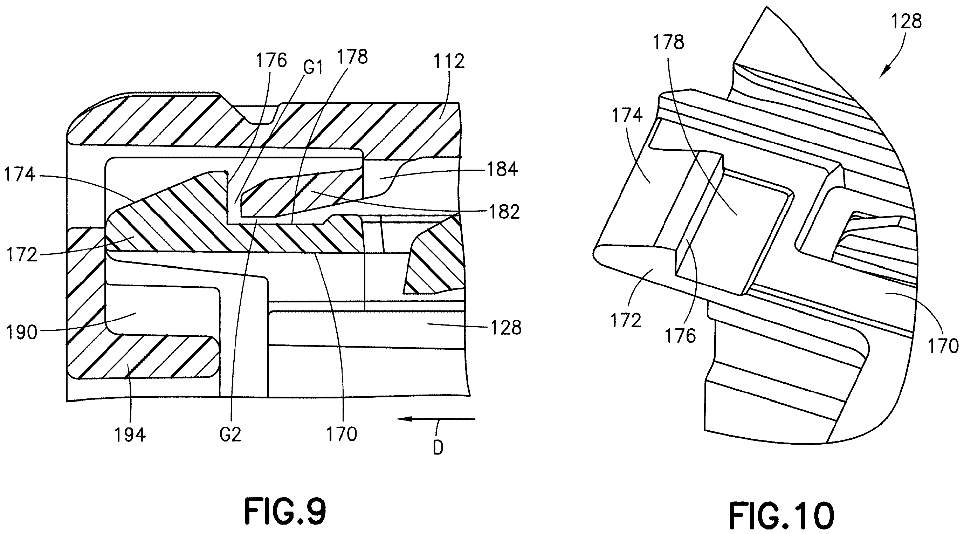

Referring to FIGS. 7 and 8, the cassette body 128 is generally U-shaped and is configured to fit within the lower housing shell 112 and receive the syringe 116 and various other components as described herein in connection with FIGS. 1-4. The cassette body 128 includes opposing sidewalls 170 which define the outer profile of the cassette body 128 and include mating features for various other components of the device 100. A distal end of each sidewall 170 includes a cassette body clip 172 configured for attachment to a corresponding lower shell clip 182 (see FIGS. 9 and 11-15) of the lower housing shell 112. Connection of the cassette body clips 172 to the corresponding lower shell clips 182 secures the cassette body 128 to the lower housing shell 112 and ensures a strong, tamper-proof assembly.

The cassette body 128 includes a needle cover clip 364 that engage the needle cover 124 to restrict the axial movement of the needle cover 124 relative to the cassette body 128. The cassette body 128 further includes motor body ribs 368 and upper housing shell ribs 370, which are configured to engage corresponding portions of the motor body 159 and the lower housing shell 112, respectively, to aid in the assembly of the device 100. The cassette body 128 also includes syringe holder stops 372, which are configured to engage portions of the syringe holder 118 to limit the axial movement of the syringe holder 118 relative to the cassette body 128.

Referring now to FIGS. 9-11, each of the cassette body clips 172 includes a ramped leading face 174 and an orthogonal proximal face 176. The ramped leading face 174 is configured to engage the corresponding lower shell clip 182 during assembly of the cassette body 128 into the lower housing shell 112. The orthogonal proximal face 176 extends outward from the sidewall 170 in a direction substantially perpendicular to a longitudinal axis of the device 100, and is configured to capture the corresponding lower shell clip 182 when the cassette body 128 is fully inserted into the lower housing shell 112. The sidewall 170 may define a pocket 178 proximally of the orthogonal proximal face 176 into which the corresponding lower shell clip 182 at least partially extends when the cassette body 128 is fully inserted into the lower housing shell 112.

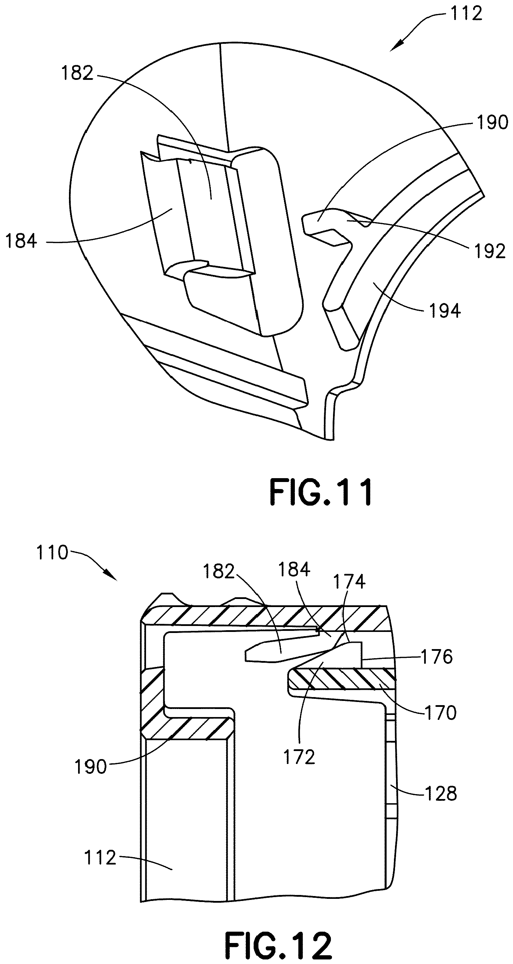

With continued reference to FIG. 9 and 11, each lower shell clip 182 extends inwards from the lower housing shell 112 toward the cassette body 128. The lower shell clip 182 includes a hinge 184 (e.g. a living hinge) integrally formed with the lower housing shell 112 that allows the lower shell clip 182 to deflect away from the cassette body 128 in response to an outwardly directed force. The lower shell clip 182 extends distally from the hinge 184 and rotates about the hinge 184 to provide clearance for insertion of the cassette body 128 into the lower housing shell 112. The lower housing shell 112 may further include a rigid wall 190 configured to limit inward deflection of the sidewall 170 of the cassette body 128, for example when the cassette body clip 172 engages the lower shell clip 182 during attempted dismantling of the device 100. The rigid wall 190 may extend proximally from a distal face of the lower housing shell 112, and the rigid wall 190 may be substantially rigid to resist deflect under load from the cassette body clip 172. The rigid wall 190 may include at least one gusset 192 connecting the rigid wall 190 to other structure of the lower housing shell 112. For example, the at least one gusset 192 may connect the rigid wall 190 to an orifice wall 194 at least partially surrounding the needle cover 124 (see FIG. 2). With the cassette body 128 assembled with the lower housing shell 112, a gap G1 is defined between the orthogonal proximal face 176 of the cassette body clip 172 and a distal end of the lower shell clip 182. Also with the cassette body 128 assembled with the lower housing shell 112, in non-limiting embodiments a gap G2 is defined between the pocket 178 and the lower shell clip 182, optionally to reduce stress on the components of drug delivery device 100.

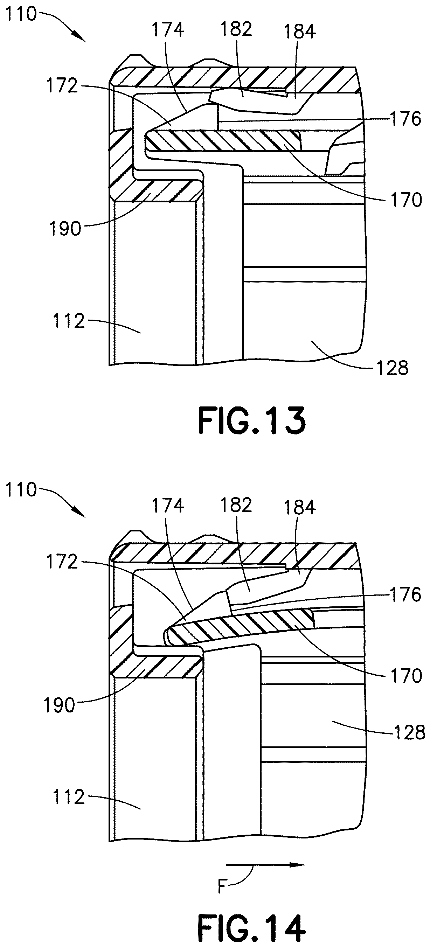

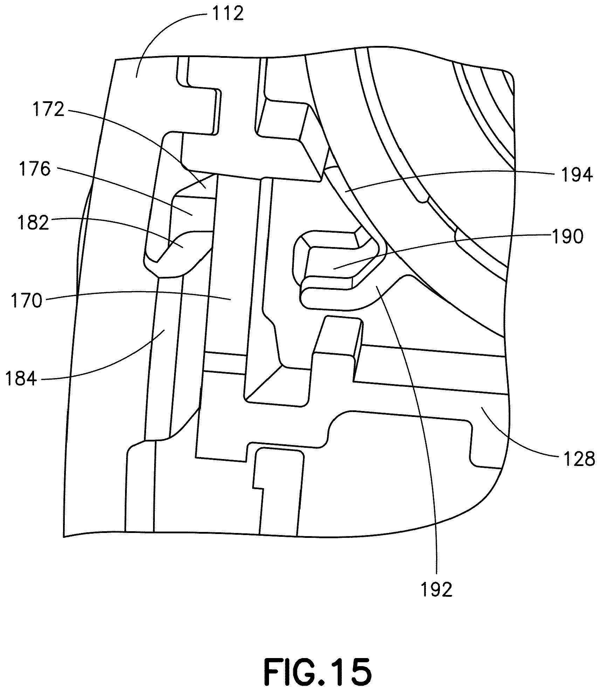

Referring now to FIGS. 12 and 13, the lower housing shell 112 and the cassette body 128 are shown during progressive stages of assembly of the first subassembly 110. As shown in FIG. 12, the cassette body 128 is inserted into the lower housing shell 112 until the ramped leading face 174 of the cassette body clip 172 contacts the lower shell clip 182. As shown in FIG. 13, continued advancement of the cassette body 128 into the lower housing shell 112 causes the ramped leading face 174 of the cassette body clip 172 to deflect the lower shell clip 182 outward, allowing the cassette body clip 172 to slide past the lower shell clip 182. The sidewall 170 of the cassette body 128 may also deflect inward to allow the cassette body clip 172 to slide past the cassette body clip 172. However, the cassette body 128 and the lower housing shell 112 are designed such that lower shell clip 182 is more deflectable (i.e. less rigid) than the cassette body clip 172, such deflection occurs predominantly in the lower shell clip 182. Once the cassette body clip 172 clears the lower shell clip 182, the lower shell clip 182 returns to its undeflected position, as shown in FIGS. 9 and 15. At this stage, the cassette body 128 is fully assembled within the lower housing shell 112. During insertion of the cassette body 128 into the lower housing shell 112, the cassette body clip 172 does not contact the rigid wall 190 of the lower housing shell 112.

Referring now to FIG. 14, withdrawal of the cassette body 128 from the lower housing shell 112 is prevented by engagement of the orthogonal proximal face 176 of the cassette body clip 172 with the distal end of the lower shell clip 182. As shown in FIG. 14, attempting to withdraw the cassette body 128 from the lower housing shell 112 - i.e., with a withdrawal force F being applied to the cassette body 128 - causes the lower shell clip 182 to deflect the cassette body clip 172 inward until the cassette body clip 172 contacts the rigid wall 190. The rigid wall 190 prohibits further inward deflection of the cassette body clip 172, while engagement of the lower shell clip 182 to the orthogonal proximal face 176 of the cassette body clip 172 prevents axial withdrawal of the cassette body 128 from the lower housing shell 112. The angle of the orthogonal proximal face 176, being substantially perpendicular to the withdrawal force F applied to the cassette body 128, causes the cassette body clip 172 to bind against the lower shell clip 182. In addition, there is no draft angle or ramp between the orthogonal proximal face 176 and the lower shell clip 182, so the cassette body clip 172 is not induced to slide out of contact with the lower shell clip 182. As such, separation of the cassette body 128 from the lower housing shell 112 is not possible without destroying the device 100.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A drug delivery device comprising:
an upper housing shell;
a lower housing shell;
a syringe comprising a barrel, a stopper, and a needle, at least a portion of the syringe positioned within the lower housing shell;
a drive assembly comprising a plunger body configured to move the stopper within the barrel upon actuation of the drive assembly;
a cassette body configured to receive the syringe; and
a motor body configured to engage the cassette body,
wherein the cassette body comprises:
a pair of sidewalls; and
at least one cassette body clip extending from the sidewalls and configured to engage a lower shell clip of the lower shell housing.

2. The drug delivery device of claim 1, wherein the at least one cassette body clip comprises an orthogonal proximal face extending outward from the sidewall in a direction substantially perpendicular to a longitudinal axis of the drug delivery device.

3. The drug delivery device of claim 1 or 2, wherein the at least one cassette body clip comprises a ramped leading face configured to engage the lower shell clip during assembly of the cassette body into the lower housing shell.

4. The drug delivery device of any of claims 1-3, wherein each of the sidewalls defines a pocket proximally of the at least one cassette body clip into which the lower shell clip at least partially extends when the cassette body is fully inserted into the lower housing shell.

5. The drug delivery device of any of claims 1-4, wherein the lower shell clip extends inward from the lower housing shell toward the cassette body.

6. The drug delivery device of any of claims 1-5, wherein the lower shell clip comprises a hinge integrally formed with the lower housing shell.

7. The drug delivery device of claim 6, wherein the lower shell clip extends distally from the hinge and is rotatable about the hinge.

8. The drug delivery device of claim 2, wherein the lower shell clip is configured to engage the orthogonal proximal face to prevent withdrawal of the cassette body from the lower housing shell.

9. The drug delivery device of any of claims 1-8, wherein the lower housing shell comprises a rigid wall configured to limit inward deflection of the sidewall of the cassette body.

10. The drug delivery device of claim 9, wherein, with a withdrawal force is applied to the cassette body, the lower shell clip is configured to deflect the cassette body clip inward until the cassette body clip contacts the rigid wall.

11. The drug delivery device of claim 9 or 10, wherein the at least one cassette body clip is configured to not contact the rigid wall during insertion of the cassette body into the lower housing shell.

12. The drug delivery device of any of claims 9-11, wherein the rigid wall comprises a gusset connecting the rigid wall to an orifice wall at least partially surrounding a needle shield of the syringe.

13. The drug delivery device of claim 2, wherein, with the cassette body assembled with the lower housing shell, a gap is defined between the orthogonal proximal face of the cassette body clip and a distal end of the lower shell clip.

14. The drug delivery device of claim 4, wherein, with the cassette body assembled with the lower housing shell, a gap is defined between the pocket and the lower shell clip.

15. The drug delivery device of any of claims 1-14, wherein the lower shell clip is less rigid than the cassette body clip.
